Europäisches Patentamt

European Patent Office

Office européen des brevets

(19)

(11) **EP 0 487 705 B2**

(12) # NEW EUROPEAN PATENT SPECIFICATION

(45) Date of publication and mention
of the opposition decision:
**03.03.1999 Bulletin 1999/09**

(45) Mention of the grant of the patent:
**20.09.1995 Bulletin 1995/38**

(21) Application number: **91912289.5**

(22) Date of filing: **05.06.1991**

(51) Int. Cl.$^6$: **B01D 53/00**, C02F 3/28

(86) International application number:
**PCT/NL91/00091**

(87) International publication number:
**WO 91/19558 (26.12.1991 Gazette 1991/29)**

(54) **PROCESS FOR THE REMOVAL OF HYDROGENSULPHIDE (H2S) FROM BIOGAS**

VERFAHREN ZUR ENTFERNUNG VON SCHWEFELWASSERSTOFF (H2S) AUS BIOGAS

PROCEDE D'ELIMINATION DU SULFURE D'HYDROGENE (H2S) CONTENU DANS DU BIOGAZ

(84) Designated Contracting States:
**AT BE CH DE DK ES FR GB GR IT LI LU NL SE**

(30) Priority: **15.06.1990 NL 9001369**

(43) Date of publication of application:
**03.06.1992 Bulletin 1992/23**

(73) Proprietor: **Paques B.V.**
**NL-8560 AB Balk (NL)**

(72) Inventor:
**HABETS, Leo, Hubertus, Alphonsus**
**NL-8571 RJ Harich (NL)**

(74) Representative:
**de Bruijn, Leendert C. et al**
**Nederlandsch Octrooibureau**
**P.O. Box 29720**
**2502 LS Den Haag (NL)**

(56) References cited:
DE-A- 3 307 796    DE-A- 3 423 285
FR-A- 2 484 990    NL-A- 8 602 150
US-A- 4 372 856

• **Report UM 1025 (section 10) of WRC plc, December 1989**
• **C.R. Henry, Hydrogen sulfide in sludge gas, JWPCF, February 1961, Vol. 33, No. 2, pages 136-140**
• **S.P.P. Ottengraf, TIBTECH, May 1987, Vol. 5, pp. 132-136**
• **F. Fischer et al., Biotechnologie, Supplement March 1985, pp. 30-33**

EP 0 487 705 B2

## Description

The invention relates to the removal of hydrogen sulphide ($H_2S$) from biogas.

Hydrogen sulphide is a disturbing but hardly avoidable component of biogas. A process frequently used for removing hydrogen sulphide from biogas is scrubbing the gas with an aqueous liquid having an increased pH. This increased pH can be adjusted by the addition of caustic soda or other agents. Such processes are known for example from European Patent Applications 229,587 and 331,806.

The efficiences that can be achieved with such processes vary from 50 to 99.9%, depending on the amount of hydroxide added and the capacity of the apparatus.

A drawback of scrubbers of the type is this high consumption of chemicals, resulting in high operational costs.

According to the present invention, the natural alkalinity generated during the aerobic biological purification of waste water is used instead of added alkaline chemicals (for example caustic soda).

Alkalinity is to be understood here as the total of negative ions and neutral particles that can dissociate $H_2S$.

This natural alkalinity can arise for example in the following two manners:

(a) Neutralized organic acids are converted into cell material and bicarbonate during the aerobic treatment, as follows:

$$R\text{-}COO^- + O_2 \rightarrow \text{cell material} + HCO_3^-$$

wherein R is for example an alkyl group, such as $CH_3$ or $C_2H_5$. Carbon dioxide ($CO_2$) is stripped by aeration and the carbonic acid equilibria are shifted as follows:

$$H_2O + HCO_3^- \rightarrow OH^- + H_2CO_3 \rightarrow H_2O + CO_2 \uparrow \text{, or}$$

$$HCO_3^- \rightarrow OH^- + CO_2 \uparrow$$

This causes the pH to increase.
(b) Organic acids are removed during the aerobic treatment according to the equation:

$$CH_3COOH \rightarrow CH_4 + CO_2$$

This results in a marked pH increase.

By stripping carbon dioxide during the aerobic post-treatment as indicated at (a), the pH value increases further.

The aerobically treated waste water having alkalinity obtained in a natural way is contacted with the biogas containing $H_2S$. The waste water also contains biomass. The $H_2S$ will be absorbed from the biogas into the aqueous phase.

The efficiencies that may be obtained in this way vary from 50 to 95%, depending on the water flow/gas flow ratio and the volume of the apparatus. For a $H_2S$ removal rate of 50%, a water flow/gas flow ratio of 0.1 is generally sufficient. For higher efficiencies, a ratio of at least 0.2 and in particular 0.5 or higher can be chosen, depending on the composition of the waste water and the biogas.

As a result of the charge neutrality, the amount of natural alkalinity in the form of $OH^-$ and $HCO_3^-$ (in meq) generated during aerobic treatment is basically equal to the number of meq/l of cations (such as $Na^+$, $K^+$, $Ca^{2+}$, $Mg^{2+}$, etc.) present minus the number of free anions (such as $Cl^-$, $SO_4^{2-}$, etc.) present. Thus, a high salt concentration prior to the aerobic treatment can lead to an increased alkalinity after the aerobic treatment and $CO_2$ stripping.

The great advantage of the present process is that no added chemicals are used, causing the operational costs to be low. A further advantage is that scrubbing liquid containing the $H_2S$ absorbed therein can be recycled to the aerobic treatment without difficulty and without further treatment. Another advantage is that the recycled washing liquid can serve to adjust the pH of the aerobic treatment, which may lead to further savings in chemicals.

The present process for removing $H_2S$ from biogas can be used not only at the site where the waste water is treated both anaerobically and aerobically, but also where a sludge fermentation is present in addition to an aerobic plant.

The biogas formed in the anaerobic phase usually contains, in addition to methane and other gases, 0.1 - 3% by volume of $H_2S$ and can be stripped of $H_2S$ according to the invention by scrubbing it with a water/biomass mixture. Reactions including the following occur then:

$$H_2S \rightleftarrows HS^- + H^+$$

$$H^+ + HCO_3^- \rightleftarrows H_2O + CO_2$$

The water phase containing the $H_2S$ absorbed therein is recycled to the aerobic phase, where a biological oxidation takes place according to the following equation:

$$H_2S + 2\,O_2 \rightarrow H_2SO_4$$

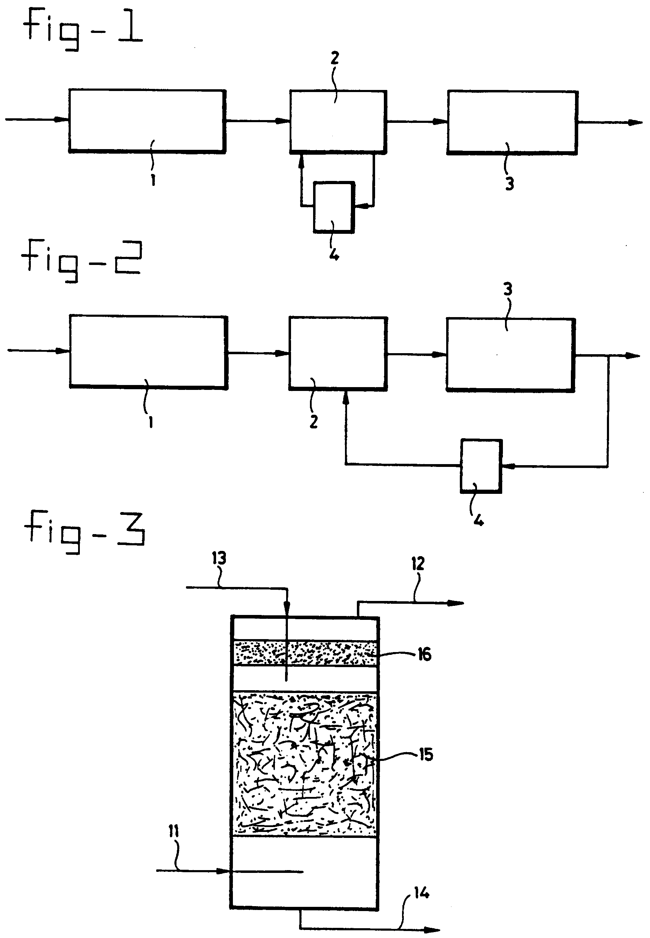

The aerobic effluent serving as scrubbing liquid can be taken from the aeration tank. The gas is scrubbed with a water/biomass mixture. This mixture containing absorbed $H_2S$ is recycled to the aeration tank. Figure 1 depicts the liquid flows according to this embodiment in diagram form. Herein (1) represents the anaerobic treatment, (2) is the aeration tank, (3) is the secondary settling and (4) is the biogas scrubber.

If clarified effluent would be used as a scrubbing liquid as shown in figure 2, wherein the reference numbers have the same meanings as in figure 1, a disadvantage would be that the secondary settling tank has a higher hydraulic load. The embodiment of figure 2 is not part of the invention.

An installation wherein the removal of $H_2S$ form biogas according to the process of the present invention can be carried out is depicted in figure 3. In addition to an inlet 11 and an outlet 12 for biogas and an inlet 13 and an outlet 14 for scrubbing liquid, this installation comprises a contact material 15 for improving the $H_2S$ transfer as well as a liquid collector 16. Inlet 13 and outlet 14 are connected with an aerobic reactor 2, which has a second inlet for waste water as shown in figure 1.

Example

The process for removing $H_2S$ from biogas has been tested in a purification plant treating waste water of a brewery.

The biogas produced in the anaerobic reactor was scrubbed with a water/biomass mixture originating from the aerobic (activated sludge) treatment of the carousel type.

| Biogas data: | |
|---|---|
| - Flow | 150-225 m3/hr |
| - $H_2S$ concentration | 0.2-0.4% |
| - $CO_2$ concentration | 28-32% |

| Carousel data: | |
|---|---|
| - Flow | 250-350 $m^3$/hr |
| - pH | 7.2-7.5 |
| - Temperature | 20-24°C |

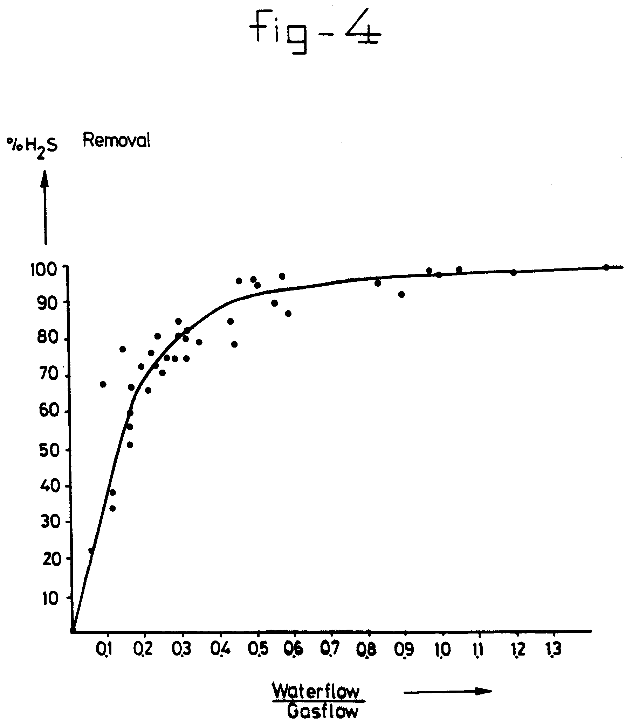

The results of the experiments are summarised in figure 4, wherein the percentage of $H_2S$ removal in the biogas scrubber is plotted as a function of the water flow/gas flow ratio.

Claims

1. Process for removing $H_2S$ from biogas by treating the gas with an alkaline scrubbing liquid in a gas scrubber, characterised in that a water/biomass mixture from an aerobic biological waste water treatment plant containing the necessary alkalinity is introduced into the gas scrubber as the scrubbing liquid in a water flow/gas flow ratio of at least 0.1, and is removed from the gas scrubber with $H_2S$ absorbed in it, and the scrubbing liquid wherein $H_2S$ is absorbed is subsequently recycled to the aerobic waste water treatment.

2. Process according to Claim 1, characterised in that a water flow/gas flow ratio of 0.2 or greater is used.

3. Purification plant for carrying out the process according to Claim 1 or 2, comprising at least a gas scrubber (4) and an aerobic waste water reactor (2), the gas scrubber (4) having a closed column equipped with a gas inlet (11) and a gas outlet (12), an inlet (13) for aerobic effluent connected with an outlet of the aerobic reactor (2), an effluent outlet (14) connected with an inlet of the aerobic reactor (2), and means (15) for contacting gas and aerobic effluent, the aerobic reactor having a second inlet for waste water.

Patentansprüche

1. Verfahren zum Entfernen von $H_2S$ aus Biogas durch Behandeln des Gases mit einer alkalischen Gaswaschflüssigkeit in einem Gaswäscher, dadurch gekennzeichnet, daß ein Wasser/Biomasse-Gemisch aus einer aeroben biologischen Abwasserbehandlungsanlage, welches die erforderliche Alkalinität enthält, in den Gaswäscher als Gaswaschflüssigkeit bei einem Wasserdurchfluß/Gasdurchfluß-Verhältnis von mindestens 0,1 eingeführt wird und aus dem Gaswäscher mit darin absorbierten $H_2S$ entfernt wird, und die Gaswaschflüssigkeit, in der $H_2S$ absorbiert ist, anschließend in die aerobe Abwasserbehandlung zurückgeführt wird.

2. Verfahren nach Anspruch 1, dadurch gekennzeichnet, daß ein Wasserdurchfluß/Gasdurchfluß-Verhältnis von 0,2 oder mehr verwendet wird.

3. Kläranlage zum Durchführen des Verfahrens nach Anspruch 1 oder 2, umfassend mindestens einen Gaswäscher (4) und einen aeroben Abwasserreaktor (2), wobei der Gaswäscher (4) eine geschlossene Säule hat, die mit einem Gaseinlaß (11) und einem Gasauslaß (12), einem Einlaß (13) für aerobes Abwasser, der mit einem Auslaß des aeroben Reaktors (2) gegebenenfalls über ein Absetzbecken (3) verbunden ist, einem Abwasserauslaß (14), der mit einem Einlaß des aeroben Reaktors (2) verbunden ist, und Mitteln (15) zum Zusammenbringen von Gas und aeroben Abwasser versehen ist,

wobei der aerobe Reaktor einen zweiten Auslaß für Abwasser hat.

## Revendications

1. Procédé d'élimination d'$H_2S$ d'un biogaz, par traitement du gaz avec un liquide de lavage alcalin dans un laveur de gaz, caractérisé en ce qu'un mélange eau/biomasse d'une usine de traitement aérobie d'eaux usées d'origine biologique, ayant l'alcalinité nécessaire, est introduit dans le laveur de gaz en tant que liquide de lavage dans un rapport débit d'eau/débit de gaz d'au moins 0,1 et est éliminé du laveur de gaz avec du $H_2S$ qu'il a absorbé, et que le liquide de lavage ayant absorbé du $H_2S$ est recyclé ensuite dans le traitement aérobie d'eaux usées.

2. Procédé selon la revendication 1, caractérisé en ce qu'on utilise un rapport débit d'eau/débit de gaz de 0,2 ou plus.

3. Usine de purification pour la mise en oeuvre du procédé selon la revendication 1 ou 2, comprenant au moins un laveur de gaz (4) et un réacteur aérobie de traitement d'eaux usées (2), le laveur de gaz (4) comprenant une colonne fermée munie d'une arrivée de gaz (11) et d'une sortie de gaz (12), une arrivée (13) pour effluent aérobie raccordée à une sortie du réacteur aérobie (2), éventuellement par l'intermédiaire d'un décanteur (3), une sortie d'effluent (14) raccordée à une entrée du réacteur aérobie (2) et des moyens (15) pour mettre en contact un gaz et un effluent aérobie, le réacteur aérobie ayant une deuxième arrivée pour des eaux usées.

fig-1

fig-2

fig-3

# fig-4